# EUROPEAN PATENT SPECIFICATION

(11) **EP 1 239 894 B1**
(45) Date of publication and mention of the grant of the patent: **09.03.2005**
(21) Application number: 00984653.6
(22) Date of filing: 20.12.2000
(51) Int. Cl.: A61L 24/10, A61L 26/00

(54) **BONE GENERATING PRODUCT**
KNOCHENBILDENDES MATERIAL
PRODUIT POUR GENERER DES OS

(30) Priority: 22.12.1999 US 469302; 16.08.2000 WO PCT/BE00/00094
(43) Date of publication of application: 18.09.2002
(73) Proprietor: Henogen S.A., 6041 Charleroi (BE)
(72) Inventor: PHILIPPART, Pierre, B-1070 Brussels (BE); BRASSEUR, Michèle, B-1082 Brussels (BE)
(74) Representative: Van Malderen, Joelle
(86) International application number: PCT/BE2000/000152
(87) International publication number: WO 2001/045760

(56) References cited:
- WO-A-97/29792
- WO-A-99/45938
- US-A- 4 427 650
- US-A- 5 266 624
- US-A- 5 733 545
- US-A- 5 883 078

## Description

### ABSTRACT OF THE DISCLOSURE

The bone generating product comprises a coagulated matrix of platelet rich plasma with a recombinant compound for generating thrombin which is thromboplastin in presence of at least a phospholipid, and an effective amount of calcium containing compound dispersed in the matrix for inducing the formation of bone.

### THE PRIOR ART

Many researches have been made for the preparation of bone substitute or implant.

For the preparation of bone substitute or implant, it is known to treat human bone by chemicals for destroying prions. The so treated human bone acts as a porous matrix suitable for the growth of cells after its implant.

It has also been proposed to prepare artificial matrix or sponge from collagen containing material and to use said matrix or sponge as bone substitute.

Example 4 of US 5,733,545 discloses the preparation of clot from a mixture containing a plasma-buffy coat concentrate and ground dry bone or from a plasma-buffy coat concentrate and CaCl₂, said latter compound being used for ensuring the coagulation of the mixture. In said example 4, it is stated that the chelation of the plasma-buffy coat concentrate containing ground dry bone is possibly due to the presence of calcium from the solid bone. In said example, it is clearly stipulated that the use of thrombin is a cause of patient complications.

However, the bone substitute obtained by mixing a plasma-buffy coat concentrate and ground dry bone was not suitable for the bone generation.

It has now been found a bone generating product, i.e. a product when implanted in a patient, allows a rapid cell colonization and the generation of bone. For obtaining a generation of bone, the bone generating product has to be prepared by using platelet rich plasma, a recombinant thrombin generating product which is thromboplastin, at least a phospholipid and an effective amount of calcium containing compound dispersed in the matrix for inducing the formation of bone. The recombinant tissue factor with or in presence of a phospholipid acts as a recombinant thrombin generating product, but also as a means for degranulocyte platelet and as a means for liberating growth factor present in the platelet. Preferably, at least a part of the calcium containing compound is made from bone particles. For example, at least 30%, preferably at least 50% by weight of the calcium containing compound is made from bone particles, preferably of not denatured bone particles. The presence of the bone particles in the bone generating product of the invention is considered as improving the generation of bone, as said bone particles contain bone morphogenic proteins as well growth factors for directing the tissue factor to produce bone. It is assumed that the excellent bone generation obtained by implanting the bone generating product of the invention to patient is due to the presence of the various factors (growth factors, etc.) present in the platelet rich plasma and of calcium containing compound(s) (preferably bone particles). The presence of recombinant tissue factor is also preferred. It is assumed that the recombinant tissue factor induces a protein containing matrix, factor suitable for inducing and accelerating the formation of bone in presence of calcium containing compound. When using substantially only platelet rich plasma, bone particles and a recombinant thrombin generating compound, it is assumed that the different factors present in the product of the invention act substantially as in the body (the ratio of one factor with respect to another being substantially equal to the said ratio in the body), whereby improving the generation of bone.

### BRIEF DESCRIPTION OF THE INVENTION

The bone generating product of the invention comprises a coagulated matrix of platelet rich plasma having a platelet concentration of 1,500,000 to 2,000,000 platelets per microliter, with thromboplastin, said matrix comprising at least a phospholipid and an effective amount of calcium containing compound for generating bone dispersed in the matrix. Preferably, the calcium containing compound is bone particles, possibly mixed with other calcium containing compound. Preferably, at least 30% by weight, advantageously at least 50% by weight of the calcium containing compound consists of bone particles. Examples of calcium containing compounds are : CaCl₂, β-tricalcium phosphate, bone particles (denatured bone), bone particles (not denatured bone), apatite, aspidine, calcium sulfate, calcium carbonate, hydroxy apatite, hydroxy apatite (from coral reef), calcium gluconolactate, calcium gluconate, calcium lactate, calcium glutoniate and mixtures thereof.

The recombinant compound for generating thrombin is a recombinant thromboplastine.
Preferably, the bone generating product of the invention comprises two or more than two different phospholipids.

According to an advantageous embodiment, the recombinant compound for generating thrombin is combined with one, preferably at least two different phospholipids.

According to an preferred embodiment, the recombinant compound for generating thrombin is combined with at least a phospholipid selected from the group consisting of phosphatidylserine, derivatives thereof, phosphatidylserine having at least one fatty acid side chain, phosphatidylcboline, derivatives thereof, phosphatidylcholine having at least one fatty acid side chain, and mixtures thereof. Preferably, the recombinant compound for generating thrombin is combined with at least a phospholipid selected from the group consisting of phosphatidylserine, derivatives thereof, phosphatidylserine having at least one fatty acid side chain, phosphatidylcholine, derivatives thereof, phosphatidylcholine having at least one fatty acid side chain, and mixtures thereof, the fatty acid side chain being selected from the group consisting of fatty acid chains with at least one double bond and with 6 to 24 carbon atoms, most preferably with 16 to 18 carbon atoms.

According to a most preferred embodiment, the recombinant compound for generating thrombin is combined with a mixture of at least two phospholipids, a first phospholipid being selected from the group consisting of phosphatidylserine, derivatives thereof, phosphatidylserine having at least one fatty acid side chain, and mixtures therof, the fatty acid side chain of the phosphatidylserine having at least one fatty acid side chain being selected from the group consisting of fatty acid chains with at least one double bond and with 6 to 24 carbon atoms, preferably 16 to 18, while the second phospholipid is selected from the group consisting of phosphatidylcholine, derivatives thereof, phosphatidylcholine having at least one fatty acid side chain, and mixtures thereof, the fatty acid side chain of the phosphatidycholine having at least one fatty acid side chain being selected from the group consisting of fatty acid chains with at least one double bond and with 6 to 24 carbon atoms.

In the bone generating product of the invention, the bone particles are preferably bone particles selected from the group consisting of craniofacial bone particles, iliac bone particles and mixture thereof. Preferably, the bone particles are particles of not denatured bones. Advantageously, the bone particles have an average particle size comprised between 0.5 mm and 5mm, preferably comprised between 0.5 and 3mm, most preferably about 1 mm (average in weight). The bone particles have for example the form of chips or flakes having an average particle size comprised between 0.5 mm and 5mm, preferably comprised between 0.5 and 3mm, most preferably about I mm (average in weight). According to a possible embodiment, the bone particles consist of a mixture of denatured bone particles (for example bone particles prepared by grinding a bone that has been treated by chemical(s), by irradiation, etc. for rendering it prion free.) and of not denatured bone particles. When using some denatured bone particles, the said particles of denatured bone can have a particle size lower than 0.5mm, as said denatured bone particles are used for adding some calcium to the product.

The bone generating product of the invention comprises for example from 5% to 50% by volume of bone particles, advantageously from 10 to 40%, preferably from 20 to 30% by volume of bone particles. The bone particles forms preferably more than 90% by weight of the calcium containing compound present in the bone generating product of the invention.

The coagulated matrix is a coagulated matrix of platelet rich plasma having a platelet concentration of 1,500,000 to 2,000,000 platelets per microlitre of the matrix forming agents, and preferably 0.05 to 0.4 µg thromboplastine in dry form per microlitre of the matrix forming agents. The platelet concentration is a concentration adapted for ensuring the viability of the platelets at 37°C.

In order to avoid as much as possible complication and in order to improve as much as possible the graft of the bone generated on the natural bone of a patient, the platelet rich plasma used is prepared from the plasma of the patient and/or from a plasma hystocompatible with the patient (i.e. immunologic hystocompatibility ), while the bone particles are prepared from a bone of the patient and/or from bone(s) hystocompatible with the patient (i.e. immuno hystocompatibility).

The bone generating product of the invention can possibly contain further components or additives, such as growth factor (superfamily BTGF and family of BMP, such as BMP-1, etc.), gene coding BMP and/or BTGF, steric factors, calcium containing compounds, drugs, fatty acids, antibiotics or mixtures of antibiotics (preferably compound(s) having an anti osteoclasts effect, such as antibiotics of the tetracyclin group, Vibramycin ®, Doxycycline ®, minocycline, minocin ® (Wyeth-Lederlee), and mixtures of compound(s) having an anti osteoclasts effect with another antibiotic(s), such as macrolide, penicillin based compounds, etc.), bactericide, virucide, fibrinogene, compounds inducing the formation of a matrix, buffer, zwitterionic buffer system at physiological pH, etc. and mixtures of said compounds or additives.

According to a detail of a preferred embodiment, the bone generating product contains from 0.001 to 10% by weight antibiotic or antibiotics (calculated in its dry form), advantageously from 0.01% to 5% by weight, preferably from 0.02 to 1%, for example from 0.05 to 0.4% by weight. The antibiotic is advantageously selected from the group consisting of antibiotics having an anti osteoclasts effect (more specifically antibiotics of the tetracyclin group, Vibramycin ®, Doxycycline ®, minocycline, minocin ® (Wyeth-Lederlee)), mixtures of antibiotics having an anti osteoclasts effect, and mixtures of one or more antibiotics having an anti osteoclasts effect with one or more other antibiotic(s) (preferably macrolide, penicillin based compounds, etc. and mixtures thereof).

Before its gelling, the bone generating product has advantageously a pH substantially equal to the physiological pH, for example a pH comprised between 6.5 and 8, preferably about 7-7.5, pH measured at 37°C.

The invention relates also to a method for the preparation of a bone generating product comprising a coagulated matrix of platelet rich plasma with a recombinant compound for generating thrombin, and bone particles dispersed in the said matrix, in which
- a substantially homogeneous mixture is formed by mixing of platelet rich plasma with an effective amount of calcium containing compound(s) for inducing the bone generation when adding to the mixture a recombinant thrombin generating compound and a phospholipid,
- a recombinant thrombin generating compound and at least one phospholipid are added and mixed to the mixture of bone particles and platelet rich plasma, and
- the mixture recombinant thrombin generating compound, platelet rich plasma, phospholipid and bone particles is kept under conditions for ensuring a coagulation of the platelet rich plasma and the formation of a bone generating matrix.

Preferably, the coagulation is carried out in presence of oxygen and substantially without stirring. The said coagulation is most preferably carried out at a temperature comprised between 35°C and 40°C, more specifically at a temperature of about 37°C.

In the process of the invention, the recombinant compound for generating thrombin used for the coagulation is recombinant thromboplastine.

Advantageously, at least two different phospholipids are added to the mixture selected from the group consisting of mixture of recombinant thrombin generating compound, platelet rich plasma and bone particles, and mixture of platelet rich plasma and bone particles, said addition being preferably carried out when adding the recombinant thrombin generating compound.

In the process of the invention, the recombinant thrombin generating compound is advantageously combined with phospholipid, preferably with phospholipids, the said compound combined with phospholipid(s) having advantageously the form of a lyophilized product, such as a lyophilized cake, powder or granules.

According to a preferred method of the invention, the recombinant compound for generating thrombin is combined with at least a phospholipid selected from the group consisting of phosphatidylserine, derivatives thereof, phosphatidylserine having at least one fatty acid side chain, phosphatidylcholine, derivatives thereof, phosphatidylcholine having at least one fatty acid side chain, and mixtures thereof, the fatty acid side chain being advantageously selected from the group consisting of fatty acid chains with at least one double bond and with 6 to 24 carbon atoms, preferably with 16-18 carbon atoms.
According to a most preferred embodiment of the process, the recombinant compound for generating thrombin is combined with a mixture of at least two phospholipids, a first phospholipid being selected from the group consisting of phosphatidylserine, derivatives thereof, phosphatidylserine having at least one fatty acid side chain, and mixtures therof, the fatty acid side chain of the phosphatidylserine having at least one fatty acid side chain being selected from the group consisting of fatty acid chains with at least one double bond and with 6 to 24 carbon atomswhile the second phospholipid is selected from the group consisting of phosphatidylcholine, derivatives thereof, phosphatidylcholine having at least one fatty acid side chain, and mixtures thereof, the fatty acid side chain of the phosphatidycholine having at least one fatty acid side chain being selected from the group consisting of fatty acid chains with at least one double bond and with 6 to 24 carbon atoms.

Preferably, at least a part of the calcium containing compound(s) is formed by bone particles, advantageously bone particles selected from the group consisting of craniofacial bone particles, iliac bone particles and mixture thereof. The bone particles are advantageously particles of not denatured bones. Said bone particles can possibly consist of a mixture of not denatured bone particles and denatured bone particles. The bone particles have advantageously an average (by weight) particle size comprised between 0.5 mm and 5mm, preferably comprised between 0.5 and 3mm, most preferably of about 1 mm.

In the method of the invention, the amount of bone particles added to the platelet rich plasma corresponds for example to about 5% to 50% by volume, advantageously from 10 to 40%, preferably from 20% to 30% by volume of the mixture platelet rich plasma and bone particles.

The platelet rich plasma used in the method of the invention has a platelet concentration of 1,500,000 to 2,000,000 platelets per microlitre of the matrix forming agents.
The mixture platelet rich plasma, bone particles and recombinant thrombin generating compound has a platelet concentration of 1,500,000 to 2,000,000 platelets per microlitre of the mixture without bone particles and contains 0.05 to 0.4 µg thromboplastine in dry form per microlitre of the mixture without bone particles.

According to a preferred method of the invention suitable for the preparation of a bone generating product for a patient, the platelet rich plasma is prepared from the plasma of the patient and/or from a plasma hystocompatible with the patient and in which the bone particles are prepared from a bone of the patient and/or from a bone hystocompatible with the patient.

According to a detail of a preferred method of the invention, the coagulation of the platelet rich plasma is carried out in presence of at least one antibiotic and/or at least one antibiotic is added to the mixture after the coagulation of the platelet rich plasma. The antibiotic or mixture of antibiotics can possibly be added to the bone particles and/or to the bone before its grinding and/or to the recombinant compound that generates thrombin and/or to a phospholipid. Preferably, an antibiotic or a mixture of antibiotics is mixed with the recombinant compound for generating thrombin (recombinant compound that generates thrombin), preferably with a recombinant thromboplastine.

According to a detail of a preferred embodiment, the amount of antibiotic or antibiotics added to the bone generating product or used during the coagulation of the platelet rich plasma bone generating product contains from 0.001 to 10% by weight antibiotic or antibiotics (calculated in its dry forms), advantageously from 0.01% to 5% by weight, preferably from 0.02 to 1%, for example from 0.05% to 0.4% by weight, more specifically from 0.2 to 0.3%. The antibiotic is advantageously selected from the group consisting of antibiotics having an anti osteoclasts effect (more specifically antibiotics of the tetracyclin group, Vibramycin ®, Doxycycline ®, minocycline, minocin ® (Wyeth-Lederlee)), mixtures of antibiotics having an anti osteoclasts effect, and mixtures of one or more antibiotics having an anti osteoclasts effect with one or more other antibiotic(s) (preferably macrolide, penicillin based compounds, etc. and mixtures thereof).

Most preferably, at least one antibiotic is added to the mixture containing at least platelet rich plasma and calcium containing compound, before adding the recombinant compound that generates thrombin, but advantageously when adding the recombinant compound that generates thrombin.

The invention relates also to the use of a recombinant compound for generating thrombin in mixture with at least one phospholipid for the preparation of a bone generating product from a mixture of a platelet rich plasma and an effective amount of calcium containing compound for inducing bone generation.

A further object of the invention is a mixture containing a recombinant compound for generating thrombin, at least one phospholipid, and a calcium containing compound, the weight ratio calcium from the calcium containing compound /recombinant compound for generating thrombin being greater than 0.5, advantageously greater than 2.
The said has advantageously the form of a dry powder.
The calcium containing compound is preferably selected from the group consisting of calcium containing salts, β- tricalcium phosphate, particles of denatured bone and mixtures thereof.

Still a further object of the invention is a mixture containing a recombinant compound for generating thrombin, at least one phospholipid and at least one antibiotic. The weight ratio antibiotic(s) (as dry matter) present in the mixture/recombinant compound for generating thrombin (as dry matter) present in the mixture is advantageously greater than 1:1, preferably greater than 3:1, most preferably greater than 5:1 and more specifically greater than 10:1. For example the said ratio is comprised between 5:1 and 50:1, more specifically between 10:1 and 25:1.

The antibiotic is advantageously selected from the group consisting of antibiotics having an anti osteoclasts effect (more specifically antibiotics of the tetracyclin group, Vibramycin ®, Doxycycline ®, minocycline, minocin ® (Wyeth-Lederlee)), mixtures of antibiotics having an anti osteoclasts effect, and mixtures of one or more antibiotics having an anti osteoclasts effect with one or more other antibiotic(s) (preferably macrolide, penicillin based compounds, etc. and mixtures thereof).

The recombinant compound that generates thrombin is advantageously a recombinant thromboplastine.

The invention relates also to a method for the preparation of a sealant, in which a fibrinogen containing solution is contacted, preferably mixed, with a recombinant compound for generating thrombin in presence of at least a phospholipid.
Advantageously, the recombinant compound for generating thrombin is a recombinant thromboplastine.

Advantageously, a gel is formed by contacting the fibrinogen containing solution with a recombinant compound for generating thrombin in presence of at least a phospholipid, at least an antibiotic and at least an effective amount of a buffer, so that the gel is formed at a pH kept between 6 and 8, advantageously between 7 and 7.5.

Preferably, a buffered solution containing the antibiotic(s) and the buffer agent(s) is prepared and contacted with the fibrinogen containing solution. The pH of said buffered solution is advantageously comprised between 6 and 8, most preferably between 7 and 7.5. The buffered solution may possibly, but advantageously, contain one or more recombinant compound for generating thrombin and/or one or more phospholipids.

Possible buffers are for example TRIS buffer, solution of Ringé, sodium bicarbonate, and mixture thereof.

Preferably, the fibrinogen containing solution is contacted with a recombinant compound for generating thrombin in presence of at least two different phospholipids.

Most preferably, the fibrinogen containing solution is contacted with a recombinant compound for generating thrombin in presence of at least one phospholipid selected from the group consisting of phosphatidylserine, derivatives thereof, phosphatidylserine having at least one fatty acid side chain, phosphatidylcholine, derivatives thereof, and phosphatidylcholine having at least one fatty acid side chain, preferably at least two phospholipids selected from said group.
The fatty acid side chain of phosphatidylcholine is advantageously selected from the group consisting of fatty acid chains with at least one double bond and with 6 to 24 carbon atoms, preferably with 16 to 18 carbon atoms.

Before the formation of the sealant, the mixed solutions have advantageously a pH substantially equal to the physiological pH, for example a pH comprised between 6.5 and 8, preferably about 7-7.5, pH measured at 37°C.

Advantageously, a platelet rich plasma is used as fibrinogen containing solution. The platelet rich plasma has a platelet concentration of 1,500,000 to 2,000,000 platelets per microlitre.
Preferably, the platelet rich plasma having a platelet concentration of 1,500,000 to 2,000,000 platelets per microlitre, while from 0.05 to 0.4 µg thromboplastine in dry form per microlitre and from 0.01 to 4 µg (advantageously from 0.1 to 0.4 µg, preferably from 0.2 to 0.3 µg) antibiotic(s) (as dry matter) per microlitre are contacted with the platelet rich plasma in presence of an effective amount of buffer agent(s) for regulating the pH between 6 and 8, advantageously between 7 and 7.5 during the gelling.

When using a platelet rich plasma, in order to avoid as much as possible complication and in order to improve as much as possible the graft of the sealant in a patient, the platelet rich plasma used is prepared from the plasma of the patient and/or from a plasma hystocompatible with the patient (i.e. immunologic hystocompatibility).

The fibrinogen containing solution, preferably the platelet rich plasma, is advantageously contacted with at least a reconibinant compound for generating thrombin in presence of at least one, preferably two different phospholipids, and in presence of at least an antibiotic.

Preferably, the fibrinogen containing solution, preferably the platelet rich plasma, is contacted with a solution containing at least a recombinant compound for generating thrombin and at least one, preferably two different phospholipids.

When using one or more antibiotics, the amount of antibiotic(s) used is advantageously such that the sealant contains from 0.001 to 10% by weight antibiotic or antibiotics, advantageously from 0.01% to 5% by weight, preferably from 0.02 to 1%, most preferably from 0.05 to 0.4%. The weight ratio antibiotic(s) (calculated as dry matter) present in the sealant mixture / recombinant compound for generating thrombin used in the sealant mixture is advantageously greater than 1:1, preferably greater than 3:1, most preferably greater than 5: and more specifically greater than 10:1. For example the said ratio is comprised between 5:1 and 50:1, more specifically between 10:1 and 25:1.

The antibiotic is advantageously selected from the group consisting of antibiotics having an anti osteoclasts effect (more specifically antibiotics of the tetracyclin group, Vibramycin ®, Doxycycline ®, minocycline, minocin ® (Wyeth-Lederlee)), mixtures of antibiotics having an anti osteoclasts effect, and mixtures of one or more antibiotics having an anti osteoclasts effect with one or more other antibiotic(s) (preferably macrolide, penicillin based compounds, etc. and mixtures thereof):
The invention relates also a kit for the preparation of a sealant according to the invention, said kit comprising:
   - a first vial containing a platelet rich plasma having a platelet concentration of 1,500,000 to 2,000,000 platelets per microliter;
   - a second vial containing a recombinant thromboplastin, preferably a solution containing said recombinant compound;
   - possibly, a third vial containing a solution to be added to the first vial and/or second vial for the preparation of a fibrinogen containing solution and/or a solution containing a recombinant compound for generating thrombin;
in which the first vial and/or the second vial and/or the third vial contains at least a phospholipid, preferably at least two different phospholipids, and in which the first vial and/or the second vial and/or the third vial (preferably the second and/or the third vial) contains at least an antibiotic.

Advantageously, the first vial and/or the second vial and/or the third vial contains at least a buffer agent. Preferably, the second vial contains at least a phospholipid and at least an antibiotic, while the third vial contain the buffer agent(s). Most preferably, the first vial contains fibrinogen containing material in a dry form, while the second vial contains a recombinant compound for generating thrombin in a dry form.

Details and characteristics of the product and the process of the invention will appear from the following description of examples.

### DESCRIPTION OF EXAMPLES

For the preparation of the said examples, the following products have been used:
PRP : platelet rich plasma of the patient to which a bone graft has to be placed. The platelet concentration of the plasma was 1,800,000 platelets per microliter of the plasma. The PRP was subjected to known usual treatments for the removal leucocyte, for obtaining a maximum proportion of life platelets, for bacteriological control, said PRP being active at least for 5 days. Prior its use, the PRP was shaken at a temperature of 37°C, the said shaking being achieved by shaking the container containing the PRP.
Thromboplastine : The thromboplastine used was a thromboplastine sold under the Trademark INNOVIN by the company DADE AG(Düdingen, Switzerland), The thromboplastine is a recombinant human tissue factor lyophilized combined with synthetic phospholipids, namely phosphatidylserine and phosphatidylcholine, said phospholipids having at least one fatty acid side chain, the fatty acid side chain being selected from the group consisting of fatty acid chains with at least one double bond and with 16-18 carbon atoms. Innovin is free of prothrombin, free of factor FVII, arid free of factor FX. Calcium is present in Innovin. Innovin is a known product for test purposes. Innovin contains also some calcium, a zwitterionic buffer system at physiological pH.
Bone particles : The bone particles have been prepared from iliac bones or craniofacial bone of the patient to whom a bone graft is needed. The fresh bone of the patient was ground in bone flakes (bone meal) having an average diameter of 1mm. The bone particles are added to the PRP just after their preparation.
Water: water used is distilled, sterilized, pyrogen free water.

### Example 1

In said example, 50ml of PRP was placed in a sterilized container. A volume of 10ml of bone particles (craniofacial provenance) was added to the PRP and mixed. The recipient is then heated under sterile conditions at 37.5°C (for example by using a water bath having a temperature of 37.5°C, the said bath containing water and 0.9% NaCl), in an oxygen containing atmosphere.

10 mg Innovin was mixed with 2 ml distilled, sterile and pyrogen free water. The mixture water + Innovin was added to the PRP + bone particles mixture kept at a temperature of 37.5°C.

After about 10minutes, without stirring, a gel is formed in the recipient, said gel being a bone generating product suitable for implant to the patient.

### Example 2

Example 1 has been repeated, except that 20mg Innovin was mixed with 2 ml distilled, sterile and pyrogen free water, and was added to the mixture PRP + bone particles.

### Examples 3 to 9

In said examples, example 1 was repeated except that the amount of reagents used was different.

| Example | 3 | 4 | 5 | 6 | 7 | 8 | 9 |
|---|---|---|---|---|---|---|---|
| PRP(ml) | 50 | 50 | 50 | 50 | 50 | 50 | 50 |
| Bone particles (ml) craniofacial | 10 | 10 | 15 | 40 | 30 | 25 | 50 |
| Innovin (mg) | 20 | 10 | 10 | 10 | 10 | 20 | 10 |
| Water (ml) | 2 | 2 | 2 | 2 | 2 | 4 | 4 |

### Examples 10 to 19

In said examples, example 1 was repeated except that the amount of reagents used was different.

| Example | 10 | 11 | 12 | 13 | 14 | 15 | 16 | 17 | 18 | 19 |
|---|---|---|---|---|---|---|---|---|---|---|
| PRP(ml) | 50 | 50 | 50 | 50 | 50 | 50 | 50 | 50 | 50 | 50 |
| Bone particles (ml) craniofacial | | | | 10 | 20 | 10 | 10 | 10 | 20 | 10 |
| Bone particles (ml) iliac | 10 | 20 | 40 | 10 | 10 | 20 | 10 | 10 | 20 | 30 |
| Innovin(mg) | 20 | 20 | 10 | 20 | 10 | 10 | 20 | 30 | 10 | 10 |
| Water (ml) | 2 | 2 | 4 | 2 | 2 | 2 | 2 | 2 | 4 | 4 |

The bone generating product of said examples 1 to 19 having the form of a gel can easily be implanted in a patient, for example in a human patient suffering a major maxillofacial atrophy. The bone generating product of the invention can easily be compacted in recesses of bones, and ca be easily be shaped.
The bone generating product of the invention was used for volunteers suffering a major maxillofacial atrophy. Sinus lift grafts and on lay graft on the maxillofacial bone have been carried out. These tests have show a bone growth or the generation of bone where the bone generating product of the invention was applied.

### Example 20

A human bone was denatured and γ-irradiated so as to be prion free. The bone was ground in particles having an average (by weight) of 0.2mm. After drying, 10g of bone particles were dry mixed with 10 mg dry INNOVIN, so as to obtain a mixture of recombinant compound for generating thrombin, phospholipid and a high level of calcium containing compound.

The so prepared mixture was then used for the preparation of a bone generating product of the invention.

The method of example 1 was repeated, except that the mixture 10 mg INNOVIN + 10g denatured bone particles was used instead of 10mg INNOVIN alone, and except that a larger amount of sterile water was used (5-10 ml), amount water adjusted so as to prepare a pasta.

### Example 21

Example 1 was repeated, except that before adding the recombinant thromboplastine, 200µg Vibramycin ® per ml mixture of PRP and bone particles was added.

### Example 22

Example 1 was repeated, except that before adding the recombinant thromboplastine, 100µg Minocycline (Minocin ®) per ml mixture of PRP and bone particles was added.

### Example 23

Example 1 was repeated, except that before adding the recombinant thromboplastine, 50µg Minocycline (Minocin ®) per ml mixture of PRP and bone particles was added.

### Example 24

Example 1 was repeated, except that before adding the recombinant thromboplastine, 20µg Minocycline (Minocin ®) per ml mixture of PRP and bone particles was added.

### Example 25

10mg of Innovin was mixed with 100mg Vibramycin ®. The presence of Vibramycin seems to improve the stability and efficiency of the Innovin.

The mixture Innovin - vibramycin ® was used as in example 1 for the preparation of the gel.

### Example 26

10 mg Innovin was mixed with 5 ml water. Thereafter, 100mg Vibramycin was added to the said aqueous mixture of Innovin. The mixture was then lyophilized so as to obtain a powder or cake containing Innovin and Vibramycin.

The lyophilized product was then used as in example 25.

As in the preparation process of Innovin, an aqueous mixture of recombinant thromboplastine and phospholipid is lyophilized, it is possible to add the said antibiotic (for example Vibramycin ) to the aqueous mixture before its lyophilization (for example before and/or after the addition of Ca⁺⁺, buffer and stabilizers to the mixture as carried out in the preparation process of Innovin).

### Examples 27 to 29

Examples 24 to 26 were repeated except that Minocin ® (Wyeth-Lederlee) was used instead of Vibramycin ®.

### Examples 30 to 59

Examples 1 to 29 have been repeated except that the recombinant tissue factor 4500L/B of American diagnostica Inc. was used instead of Innovin. The recombinant tissue factor 4.500L/B2 of American diagnostics Inc. can also be used.

### Example 60

A sealant was prepared by mixing 50 ml ofPRP with 2ml aqueous solution containing 10mg Innovin, 100mg Minocin ® and a physiologically acceptable buffer (in an amount sufficient for having a pH of about 7.2 for the aqueous solution before its mixing with the PRP, for example a solution of sodium bicarbonate). A sealant gel was so obtained.

### Example 61

50 ml PRP was placed in a chamber of a first syringe, while 2ml aqueous solution containing 10mg Innovin,100mg Minocin ® and a physiologically acceptable buffer (sodium bicarbonate) was placed in a chamber of a second syringe. The two syringes were connected to a mixer for mixing PRP with Innovin, Minocin and buffer before applying the mixture to the wound, and to a means for delivering to the mixer on a continuous manner 0.04ml Innovin solution per ml of PRP.

### Example 62

Example 61 was repeated except that 10ml of an aqueous solution containing 10mg Innovin and 200 mg Vibramycin ® and buffer agent (the pH of the solution being about 7.2) was used, and that the means delivers to the mixer on a continuous manner 0.2ml Innovin-Vibramycin solution per ml of PRP.

### Example 63

A kit for the preparation of a sealant, said kit comprising:
- a first vial containing PRP ;
- a second vial containing Innovin and Vibramycin in a dry form,
- a third vial containing sterile water and a buffer (amount sufficient for ensuring a pH of 7.2, when mixing the three vials together, preferably the second and third vials are first mixed together and the mixture thereof is mixed with the content of the first vial) for reconstituting the solution containing Innovin-Vibramycin.
Advantageously, the kit further comprises means for mixing the PRP with the reconstituted Innovin-Vibramycin solution and means for applying the mixture on the wound, for example by spraying.

### Example 64

Sealant composition of the invention and pasta (bone generating product), especially as prepared in the previous example, were used for coating an artificial bone, for example a bone having been submitted to one or more sterilization treatments.

## Claims

1. A method for the preparation of a sealant, in which a platelet rich plasma having a platelet concentration of 1.500.000 to 2.000.000 platelets per microlitre, is contacted with a recombinant thromboplastin in presence of at least one phospholipid and buffer agent.

2. The method of claim 1, in which a gel is formed by contacting a platelet rich plasma, having a platelet concentration of 1.500.000 to 2.000.000 platelets per microlitre, with a recombinant compound for generating thrombin in presence of at least a phospholipid, at least an antibiotic and at least an effective amount of buffer, so that the pH of the contacted platelet rich plasma is kept between 6 and 8, advantageously between 7 and 7.5 during the formation of the gel.

3. The method of claim 2, in which a buffered solution containing the antibiotic(s) and buffer, possibly a recombinant compound for generating thrombin and possibly a phospholipid is prepared, said buffered solution having a pH comprised between 6 and 8, preferably between 7 and 7.5 and in which the platelet rich plasma is contacted with said buffered solution.

4. The method of any one of the claims 1 to 3 in which the platelet rich plasma is contacted with a recombinant compound for generating thrombin in presence of at least two different phospholipids.

5. The method according to any one of the claims 1 to 4, in which the platelet rich plasma is contacted with a recombinant compound for generating thrombin in presence of at least one phospholipid selected from the group consisting of phosphatidylserine, derivatives thereof, phosphatidylserine having at least one fatty acid side chain, phosphatidylcholine, derivatives thereof, and phosphatidycholine having at least one fatty acid side chain, preferably at least two phospholipids selected from said group.

6. The method of claim 5, in which the fatty acid side chain of phosphatidylcholine is selected from the group consisting of fatty acid chains with at least one double bond and with 6 to 24 carbon atoms, preferably with 16 to 18 carbon atoms.

7. The method of the claims 1 to 6, wherein the platelet rich plasma is contacted with at least an antibiotic having an anti osteoclastic effect.

8. The method of claim 1 to 7, in which the platelet rich plasma, while from 0.05 to 0.4 *µ*g thromboplastine in dry form per microlitre and from 0.05 to 4 *µ*g antibiotic (s) per microlitre are contacted with the platelet rich plasma in presence of an effective amount of buffer agent(s) for regulating the pH between 6 and 8, advantageously between 7 and 7.5 during the gelling.

9. A kit for the-preparation of a sealant, said kit comprising:
- a first vial containing a platelet rich plasma having a platelet concentration of 1.500.000 to 2.000.000 platelets per microlitre;
- a second vial containing a recombinant thromboplastin, preferably a solution containing said recombinant thromboplastin;
- possibly, a third vial containing a solution to be added to the first vial and/or second vial for the preparation of a platelet rich plasma having a platelet concentration of 1.500.000 to 2.000.000 platelets per microlitre and/or a solution containing a recombinant compound for generating thrombin;
in which the first vial and/or the second vial and/or the third vial contains at least a phospholipid, preferably at least two different phospholipids, and in which the first vial and/or the second vial and/or the third vial, preferably the second vial or the third vial, contains at least an antibiotic.

10. The kit of claim 9, in which the first vial and/or the second vial and/or the third vial contains at least a buffer agent.

11. The kit of claim 10, in which the second vial contains at least a phospholipid, at least a buffer agent and at least an antibiotic.

12. The kit of claim 9, in which the first vial contains a platelet rich plasma having a platelet concentration of 1.500.000 to 2.000.000 platelets per microlitre in a dry form, while the second vial contains a recombinant compound for generating thrombin in a dry form.

13. Bone generating product comprising a coagulated matrix of platelet rich plasma having a platelet concentration of 1.500.000 to 2.000.000 platelets per microlitre of the matrix forming agent with a recombinant thromboplastin in presence of at least a phospholipid, and an effective amount of calcium containing compound dispersed in the said matrix for inducing the formation of bone.

14. The bone generating product of claim 13, in which the calcium containing compound is selected from the group consisting of bone particles, mixture of bone particles with calcium containing additives.

15. The bone generating product of claim 13, which further comprises at least two different phospholipids.

16. The bone generating product of claim 13, in which the recombinant compound for generating thrombin is combined with phospholipids.

17. The bone generating product of claim 13, in which the recombinant compound for generating thrombin is combined with at least a phospholipid selected from the group consisting of phosphatidylserine, derivatives thereof, phosphatidylserine having at least one fatty acid side chain, phosphatidylcholine, derivatives thereof, phosphatidylcholine having at least one fatty acid side chain, and mixtures thereof.

18. The bone generating product of claim 13, in which the recombinant compound for generating thrombin is combined with at least a phospholipid selected from the group consisting of phosphatidylserine, derivatives thereof, phosphatidylserine having at least one fatty acid side chain, phosphatidylcholine, derivatives thereof, phosphatidylcholine having at least one fatty acid side chain, and mixtures thereof, the fatty acid side chain being selected from the group consisting of fatty acid chains with at least one double bond and with 6 to 24 carbon atoms.

19. The bone generating product of claim 13, in which the recombinant compound for generating thrombin is combined with at least a phospholipid selected from the group consisting of phosphatidylserine, derivatives thereof, phosphatidylserine having at least one fatty acid side chain, phosphatidylcholine, derivatives thereof, phosphatidylcholine having at least one fatty acid side chain, and mixtures thereof, the fatty acid side chain being selected from the group consisting of fatty acid chains with at least one double bond and with 16 to 18 carbon atoms.

20. The bone generating product of claim 13, in which the recombinant compound for generating thrombin is combined with a mixture of at least two phospholipids, a first phospholipid being selected from the group consisting of phosphatidylserine, derivatives thereof, phosphatidylserine having at least one fatty acid side chain, and mixtures thereof, the fatty acid side chain of the phosphatidylserine having at least one fatty acid side chain being selected from the group consisting of fatty acid chains with at least one double bond and with 6 to 24 carbon atoms while the second phospholipid is selected from the group consisting of phosphatidylcholine, derivatives thereof, phosphatidylcholine having at least one fatty acid side chain, and mixtures thereof, the fatty acid side chain of the phosphatidycholine having at least one fatty acid side chain being selected from the group consisting of fatty acid chains with at least one double bond and with 6 to 24 carbon atoms.

21. The bone generating product of claim 13, in which the calcium containing compound is selected from the group consisting of bone particles, mixture of bone particles with calcium containing additives, and in which the bone particles are bone particles selected from the group consisting of craniofacial bone particles, iliac bone particles and mixtures thereof.

22. The bone generating product of claim 13, in which the calcium containing compound is selected from the group consisting of bone particles, mixture of bone particles with calcium containing additives, and in which the bone particles are particles of not denatured bones.

23. The bone generating product of claim 13, in which the bone particles have an average particle size comprised between 0.5 mm and 5 mm.

24. The bone generating product of claim 13, in which the calcium containing compound is selected from the group consisting of bone particles, mixture of bone particles with calcium containing additives, the product comprising from 15% to 50% by volume of bone particles.

25. The bone generating product of claim 13, in which the coagulated matrix is a coagulated matrix of platelet rich plasma having a platelet concentration of 1,500,000 to 2,000,000 platelets per microlitre of the matrix forming agents and 0.05 to 0.4 *µ*g thromboplastine in dry form per microlitre of the matrix forming agents.

26. The bone generating product of claim 13 for a patient, in which the platelet rich plasma is prepared from the plasma of the patient and in which the bone particles are prepared from a bone of the patient.

27. The bone generating product of claim 13, in which the coagulated matrix is associated with a bio compatible film.

28. The bone generating product of claim 13, which further contains at least an additive selected among the group consisting of growth factors, genes encoding growth factors, calcium containing compounds, drugs, fatty acids, antibiotics, bactericides, virucides, fibrinogene, compounds inducing the formation of matrixes, and mixtures thereof.

29. The bone generating product of claim 28, which contains as antibiotic at least an antibiotic having an anti osteoclastic effect.

30. A method for the preparation of a bone generating product comprising a coagulated matrix of platelet rich plasma having a platelet concentration of 1.500.000 to 2.000.000 platelets per microlitre of the matrix forming agent, with a recombinant thromboplastin, and bone particles dispersed in the said matrix, in which
- a substantially homogeneous mixture is prepared by mixing platelet rich plasma with an amount of calcium containing compound effective for inducing the generation of bone when adding a recombinant thrombin generating compound and a phospholipid,
- a recombinant thrombin generating compound and a phospholipid are added and mixed to the mixture prepared from platelet rich plasma, and
- the mixture recombinant thrombin generating compound, phospholipid, platelet rich plasma and calcium containing compound is kept under conditions for ensuring a coagulation of the platelet rich plasma and the formation of a matrix.

31. The method of claim 30, in which the calcium containing compound is selected from the group consisting of bone particles, mixture of bone particles with calcium containing additives.

32. The method of claim 30, in which the coagulation is carried out in presence of oxygen and substantially without stirring.

33. The method of claim 30, in which the calcium containing compound is selected from the group consisting of bone particles, mixture of bone particles with calcium containing additives, and in which at least one phospholipid is added to the mixture selected from the group consisting of mixture of recombinant thrombin generating compound, platelet rich plasma and bone particles, and mixture of platelet rich plasma and bone particles.

34. The method of claim 30, in which the recombinant compound for generating thrombin is combined with phospholipids.

35. The method of claim 30, in which the recombinant compound for generating thrombin is combined with at least a phospholipid selected from the group consisting of phosphatidylserine, derivatives thereof, phosphatidylserine having at least one fatty acid side chain, phosphatidylcholine, derivatives thereof, phosphatidylcholine having at least one fatty acid side chain, and mixtures thereof.

36. The method of claim 30, in which the recombinant compound for generating thrombin is combined with at least a phospholipid selected from the group consisting of phosphatidylserine, derivatives thereof, phosphatidylserine having at least one fatty acid side chain, phosphatidylcholine, derivatives thereof, phosphatidylcholine having at least one fatty acid side chain, and mixtures thereof, the fatty acid side chain being selected from the group consisting of fatty acid chains with at least one double bond and with 6 to 24 carbon atoms.

37. The method of claim 30, in which the recombinant compound for generating thrombin is combined with at least a phospholipid selected from the group consisting of phosphatidylserine, derivatives thereof, phosphatidylserine having at least one fatty acid side chain, phosphatidylcholine, derivatives thereof, phosphatidylcholine having at least one fatty acid side chain, and mixtures thereof, the fatty acid side chain being selected from the group consisting of fatty acid chains with at least one double bond and with 16 to 18 carbon atoms.

38. The method of claim 30, in which the recombinant compound for generating thrombin is combined with a mixture of at least two phospholipids, a first phospholipid being selected from the group consisting of phosphatidylserine, derivatives thereof, phosphatidylserine having at least one fatty acid side chain, and mixtures thereof, the fatty acid side chain of the phosphatidylserine having at least one fatty acid side chain being selected from the group consisting of fatty acid chains with at least one double bond and with 6 to 24 carbon atoms while the second phospholipid is selected from the group consisting of phosphatidylcholine, derivatives thereof, phosphatidylcholine having at least one fatty acid side chain, and mixtures thereof, the fatty acid side chain of the phosphatidycholine having at least one fatty acid side chain being selected from the group consisting of fatty acid chains with at least one double bond and with 6 to 24 carbon atoms.

39. The method of claim 30, in which the calcium containing compound is selected from the group consisting of bone particles, mixture of bone particles with calcium containing additives, and in which the bone particles are bone particles selected from the group consisting of craniofacial bone particles, iliac bone particles and mixture thereof.

40. The method of claim 30, in which the calcium containing compound is selected from the group consisting of bone particles, mixture of bone particles with calcium containing additives, and in which the bone particles are particles of not denatured bones.

41. The method of claim 30, in which the calcium containing compound is selected from the group consisting of bone particles, mixture of bone particles with calcium containing additives, and in which the bone particles have an average particle size comprised between 0.5 mm and 5 mm.

42. The method of claim 30, in which the calcium containing compound is selected from the group consisting of bone particles, mixture of bone particles with calcium containing additives, and in which the amount of bone particles added to the platelet rich plasma corresponds to about 15% to 50% by volume of the mixture platelet rich plasma and bone particles.

43. The method of claim 30, in which the mixture platelet rich plasma, bone particles and recombinant thrombin generating compound has a platelet concentration of 1,500,000 to 2,000,000 platelets per microlitre of the mixture without bone particles and contains 0.05 to 0.4 *µ*g thromboplastine in dry form per microlitre of the mixture without bone particles.

44. The method of claim 30, for the preparation of a bone generating product for a patient, in which the platelet rich plasma is prepared from a plasma selected from the group consisting of the plasma of the patient, a plasma hystocompatible with the patient and mixtures thereof, and in which the calcium containing compound consists essentially of bone particles prepared from at least a bone selected from the group consisting of bones of the patient, bones hystocompatible with the patient and mixtures thereof.

45. The method of claim 30, in which the coagulation of the platelet rich plasma is made in presence of an antibiotic, having preferably an anti osteoclastic effect.

46. Use of a recombinant thromboplastin in mixture with at least one phospholipid for the preparation of a bone generating product from a mixture of a platelet rich plasma, having a platelet concentration of 1.500.000 to 2.000.000 platelets per microlitre, an effective amount. of calcium containing compound for inducing bone generation, and possibly an antibiotic having preferably an anti osteoclastic effect.

## Patentansprüche

1. Ein Verfahren zur Herstellung eines Dichtungsmittels, wobei ein thrombozytenreiches Plasma mit einer Thrombozytenkonzentration von 1.500.000 bis 2.000.000 Thrombozyten pro Mikroliter mit einem rekombinanten Thromboplastin in Anwesenheit von mindestens einem Phospholipid und Pufferagens in Kontakt gebracht wird.

2. Das Verfahren gemäß Anspruch 1, wobei durch Kontakt eines thrombozytenreichen Plasmas mit einer Konzentration von 1.500.000 bis 2.000.000 Thrombozyten pro Mikroliter und einer rekombinanten Verbindung zur Erzeugung von Thrombin in Anwesenheit von zumindest einem Phospholipid, mindestens einem Antibiotikum und mindestens einer effektiven Menge an Puffer ein Gel erzeugt wird, sodass der pH-Wert des kontaktierenden thrombozytenreichen Plasmas während der Gelbildung zwischen 6 und 8 und idealerweise zwischen 7 und 7,5 gehalten wird.

3. Das Verfahren gemäß Anspruch 2, wobei eine gepufferte Lösung, welche das Antibiotikum oder die Antibiotika und den Puffer und möglicherweise eine rekombinante Verbindung zur Erzeugung von Thrombin und möglicherweise ein Phospholipid enthält, zubereitet wird, wobei die besagte Pufferlösung einen pH-Wert zwischen 6 und 8, vorzugsweise zwischen 7 und 7,5, aufweist und wobei das thrombozytenreiche Plasma mit der besagten Pufferlösung in Kontakt gebracht wird.

4. Das Verfahren gemäß einem der Ansprüche 1 bis 3, wobei das thrombozytenreiche Plasma in Anwesenheit von mindestens zwei unterschiedlichen Phospholipiden mit einer rekombinanten Verbindung in Kontakt gebracht wird.

5. Das Verfahren gemäß irgendeinem der Ansprüche 1 bis 4, wobei das thrombozytenreiche Plasma mit einer rekombinanten Verbindung zur Erzeugung von Thrombin in Kontakt gebracht wird, und dies in Anwesenheit von zumindest einem Phospholipid, das aus der Gruppe, die aus Phosphatidylserin, Derivaten davon, Phosphatidylserin mit zumindest einer Fettsäurenseitenkette, Phosphatidylcholin, Derivaten davon und Phosphatidylcholin mit zumindest einer Fettsäurenseitenkette besteht, ausgewählt wurde, wobei zumindest zwei Phospholipide aus der besagten Gruppe vorzuziehen wären.

6. Das Verfahren gemäß Anspruch 5, wobei die Fettsäurenseitenkette des Phosphatidylcholins aus der Gruppe ausgewählt wird, die aus Fettsäurenseitenketten mit zumindest einer Doppelbindung und 6 bis 24 Kohlenstoffatomen, vorzugsweise mit 16 bis 18 Kohlenstoffatomen, besteht.

7. Das Verfahren gemäß Ansprüchen 1 bis 6, wobei das thrombozytenreiche Plasma mit zumindest einem Antibiotikum in Kontakt gebracht wird, das einen antiosteoklastischen Effekt hat.

8. Das Verfahren gemäß Ansprüchen 1 bis 7, wobei 0,05 bis 0,4 *µ*g Thromboplastin in trockener Form pro Mikroliter und 0,05 bis 4 *µ*g Antibiotikum(a) pro Mikroliter mit dem thrombozytenreichen Plasma in Kontakt gebracht werden, und dies in Anwesenheit von einer effektiven Menge des Pufferagens zur Regulierung des pH-Wertes zwischen 6 und 8, wobei während der Gelierung ein pH-Wert zwischen 7 und 7,5 von Vorteil wäre.

9. Ein Bausatz zur Herstellung eines Dichtungsmittels, wobei der besagte Bausatz folgendes enthält:
- Ein erstes Fläschchen, welches ein thrombozytenreiches Plasma enthält, das eine Konzentration von 1.500.000 bis 2.000.000 Thrombozyten pro Mikroliter aufweist.
- Ein zweites Fläschchen, welches ein rekombinantes Thromboplastin enthält, vorzugsweise eine Lösung, welche das besagte rekombinante Thromboplastin enthält.
- Möglicherweise ein drittes Fläschchen, welches eine Lösung enthält, die in das erste oder das zweite Fläschchen gegeben werden kann, um ein thrombozytenreiches Plasma zu erzeugen, das über eine Thrombozytenkonzentration von 1.500.000 bis 2.000.000 pro Mikroliter verfügt und/oder eine Lösung, die eine rekombinante Verbindung zur Erzeugung von Thrombin enthält.
wobei das erste Fläschchen und/oder das zweite Fläschchen und/oder das dritte Fläschchen zumindest ein Phospholipid enthält, vorzugsweise zumindest zwei verschiedene Phospholipide, und wobei das erste Fläschchen und/oder das zweite Fläschchen und/oder das dritte Fläschchen, aber vorzugsweise das zweite oder das dritte Fläschchen, zumindest ein Antibiotikum enthält.

10. Der Bausatz gemäß Anspruch 9, wobei das erste Fläschchen und/oder das zweite Fläschchen und/oder das dritte Fläschchen zumindest ein Pufferagens enthält.

11. Der Bausatz gemäß Anspruch 10, wobei das zweite Fläschchen zumindest ein Phospholipid, zumindest ein Pufferagens und zumindest ein Antibiotikum enthält.

12. Der Bausatz gemäß Anspruch 9, wobei das erste Fläschchen ein thrombozytenreiches Plasma mit einer Thrombozytenkonzentration von 1.500.000 bis 2.000.000 Thrombozyten pro Mikroliter in trockener Form enthält, während das zweite Fläschchen eine rekombinante Verbindung zur Erzeugung von Thrombin in trockener Form enthält.

13. Ein knochenerzeugendes Produkt, welches eine koagulierte Matrize aus thrombozytenreichem Plasma mit einer Thrombozytenkonzentration von 1.500.000 bis 2.000.000 Thrombozyten pro Mikroliter des matrizebildenden Agens mit einem rekombinanten Thromboplastin in Anwesenheit von mindestens einem Phospholipid enthält, sowie eine effektive Menge einer kalziumhaltigen Verbindung, welche in der besagten Matrize aufgelöst wurde, um die Knochenbildung zu induzieren.

14. Das knochenerzeugende Produkt gemäß Anspruch 13, wobei die kalziumhaltige Verbindung aus der Gruppe ausgewählt wird, die aus Knochenpartikeln und einer Mischung aus Knochenpartikeln und kalziumhaltigen Additiven besteht.

15. Das knochenerzeugende Produkt gemäß Anspruch 13, welches weiterhin zumindest zwei verschiedene Phospholipide enthält.

16. Das knochenerzeugende Produkt gemäß Anspruch 13, in welchem die rekombinante Verbindung zur Erzeugung von Thrombin mit Phospholipiden kombiniert ist.

17. Das knochenerzeugende Produkt gemäß Anspruch 13, in welchem die rekombinante Verbindung zur Erzeugung von Thrombin mit zumindest einem Phospholipid kombiniert ist, welches aus der Gruppe ausgewählt wurde, die aus Phosphatidylserin, Derivaten davon, Phosphatidylserin mit zumindest einer Fettsäurenseitenkette, Phosphatidylcholin, Derivaten davon, Phosphatidylcholin mit zumindest einer Fettsäurenseitenkette sowie Mischungen daraus besteht.

18. Das knochenerzeugende Produkt gemäß Anspruch 13, in welchem die rekombinante Verbindung zur Erzeugung von Thrombin mit zumindest einem Phospholipid kombiniert ist, welches aus der Gruppe ausgewählt wurde, die aus Phosphatidylserin, Derivaten davon, Phosphatidylserin mit zumindest einer Fettsäurenseitenkette, Phosphatidylcholin, Derivaten davon, Phosphatidylcholin mit zumindest einer Fettsäurenseitenkette sowie Mischungen daraus besteht, wobei die Fettsäurenseitenkette aus der Gruppe ausgewählt wurde, welche aus Fettsäureseitenketten mit zumindest einer Doppelbindung und 6 bis 24 Kohlenstoffatomen besteht.

19. Das knochenerzeugende Produkt gemäß Anspruch 13, in welchem die rekombinante Verbindung zur Erzeugung von Thrombin mit zumindest einem Phospholipid kombiniert ist, welches aus der Gruppe ausgewählt wurde, die aus Phosphatidylserin, Derivaten davon, Phosphatidylserin mit zumindest einer Fettsäurenseitenkette, Phosphatidylcholin, Derivaten davon, Phosphatidylcholin mit zumindest einer Fettsäurenseitenkette sowie Mischungen daraus besteht, wobei die Fettsäurenseitenkette aus der Gruppe ausgewählt wurde, welche aus Fettsäureseitenketten mit zumindest einer Doppelbindung und mit 16 bis 18 Kohlenstoffatomen besteht.

20. Das knochenerzeugende Produkt gemäß Anspruch 13, in welchem die rekombinante Verbindung zur Erzeugung von Thrombin mit einer Mischung aus zumindest zwei Phospholipiden kombiniert ist, wobei ein erstes Phospholipid aus der Gruppe ausgewählt wurde, welche aus Phosphatidylserin, Derivaten davon, Phosphatidylserin mit zumindest einer Fettsäurenseitenkette und Mischungen daraus ausgewählt wurde, wobei die Fettsäurenseitenkette des Phosphatidylserin zumindest über eine Fettsäurenseitenkette verfügt, die aus der Gruppe ausgewählt wurde, welche aus Fettsäurenseitenketten mit zumindest einer Doppelbindung und mit 6 bis 24 Kohlenstoffatomen besteht, während das zweite Phospholipid aus der Gruppe ausgewählt wurde, die aus Phosphatidylcholin, Derivaten davon, Phosphatidylcholin mit zumindest einer Fettsäurenseitenkette und Mischungen daraus besteht, wobei die Fettsäurenseitenkette des Phosphatidylcholins zumindest über eine Fettsäurenseitenkette verfügt, die aus der Gruppe ausgewählt wurde, die aus Fettsäurenseitenkette mit zumindest einer Doppelbindung und 6 bis 24 Kohlenstoffatomen besteht.

21. Das knochenerzeugende Produkt gemäß Anspruch 13, in welchem die kalziumhaltige Verbindung aus der Gruppe ausgewählt wurde, die aus Knochenpartikeln und einer Mischung aus Knochenpartikeln mit kalziumhaltigen Additiven besteht und wobei die Knochenpartikel aus der Gruppe ausgewählt wurden, welche aus Knochenpartikeln des Schädels und des Hüftbeins und Mischungen daraus besteht.

22. Das knochenerzeugende Produkt gemäß Anspruch 13, in welchem die kalziumhaltige Verbindung aus der Gruppe ausgewählt wurde, die aus Knochenpartikeln und einer Mischung aus Knochenpartikeln mit kalziumhaltigen Additiven besteht und wobei die Knochenpartikel Partikel aus nicht denaturierten Knochen sind.

23. Das knochenerzeugende Produkt gemäß Anspruch 13, in welchem die Knochenpartikel eine durchschnittliche Partikelgröße zwischen 0,5 mm und 5 mm aufweisen.

24. Das knochenerzeugende Produkt gemäß Anspruch 13, in welchem die kalziumhaltige Verbindung aus der Gruppe ausgewählt wurde, die aus Knochenpartikeln und einer Mischung aus Knochenpartikeln mit kalziumhaltigen Additiven besteht, wobei das Produkt zwischen 15 und 50 Volumenprozent Knochenpartikel enthält.

25. Das knochenerzeugende Produkt gemäß Anspruch 13, in welchem die koagulierte Matrize eine koagulierte Matrize aus thrombozytenreichem Plasma mit einer Thrombozytenkonzentration von 1.500.000 bis 2.000.000 Thrombozyten pro Mikroliter des matrizebildenden Agens und 0,05 bis 0,4 *µ*g Thromboplastin in trockener Form pro Mikroliter des matrizebildenden Agens ist.

26. Das knochenerzeugende Produkt gemäß Anspruch 13 für einen Patienten, wobei das thrombozytenreiche Plasma aus dem Plasma des Patienten hergestellt wurde und wobei die Knochenpartikel aus einem Knochen des Patienten hergestellt wurden.

27. Das knochenerzeugende Produkt gemäß Anspruch 13, wobei die koagulierte Matrize mit einem biokompatiblen Film verbunden ist.

28. Das knochenerzeugende Produkt gemäß Anspruch 13, welches des Weiteren zumindest ein Additiv enthält, welches aus der Gruppe ausgewählt wurde, die aus Wachstumsfaktoren, Gene, die die Wachstumsfaktoren aufschlüsseln, kalziumhaltigen Verbindungen, Medikamenten, Fettsäuren, Antibiotika, Bakteriziden, Viruziden, Fibrinogenen und Verbindungen besteht, welche die Bildung von Matrizen induzieren, sowie Mischungen davon.

29. Das knochenerzeugende Produkt gemäß Anspruch 28, welches als Antibiotikum zumindest ein Antibiotikum enthält, das einen antiosteoklastischen Effekt hat.

30. Ein Verfahren zur Herstellung eines knochenerzeugenden Produkts, welches eine koagulierte Matrize aus thrombozytenreichem Plasma mit einer Konzentration von 1.500.000 bis 2.000.000 Thrombozyten pro Mikroliter des matrizebildenden Agens mit einem rekombinanten Thromboplastin enthält, wobei in der besagten Matrize Knochenpartikel dispergiert sind und wobei:
- eine im wesentlichen homogene Mischung erzeugt wird, in dem das thrombozytenreiche Plasma mit einer bestimmten Menge einer kalziumhaltigen Verbindung vermischt wird, welche die Erzeugung von Knochen induzieren kann, wenn eine rekombinante, Thrombin erzeugende Verbindung und ein Phospholipid hinzugegeben werden,
- eine Thrombin erzeugende Verbindung und ein Phospholipid zugegeben und mit der Mischung vermischt werden, die aus dem thrombozytenreichen Plasma hergestellt wurde und
- die Mischung aus der rekombinanten, Thrombin erzeugenden Verbindung, dem Phospholipid, dem thrombozytenreichen Plasma und der kalziumhaltigen Verbindung erhalten bleibt, um die Koagulation des thrombozytenreichen Plasmas und die Bildung von einer Matrize sicherzustellen.

31. Das Verfahren gemäß Anspruch 30, wobei die kalziumhaltige Verbindung aus der Gruppe ausgewählt wurde, welche aus Knochenpartikeln und einer Mischung aus Knochenpartikeln und kalziumhaltigen Additiven besteht.

32. Das Verfahren gemäß Anspruch 30, wobei die Koagulation in Anwesenheit von Sauerstoff und im wesentlichen ohne Rühren erfolgt.

33. Das Verfahren gemäß Anspruch 30, wobei die kalziumhaltige Verbindung aus der Gruppe ausgewählt wurde, welche aus Knochenpartikeln und einer Mischung aus Knochenpartikeln und kalziumhaltigen Additiven besteht und wobei zumindest ein Phospholipid zu der Mischung hinzugegeben wird, welche aus der Gruppe ausgewählt wurde, die aus einer Mischung einer rekombinanten, Thrombin erzeugenden Verbindung, thrombozytenreichem Plasma und Knochenpartikeln und einer Mischung aus thrombozytenreichem Plasma und Knochenpartikeln besteht.

34. Das Verfahren gemäß Anspruch 30, wobei die rekombinante Verbindung zur Erzeugung von Thrombin mit Phospholipiden kombiniert wird.

35. Das Verfahren gemäß Anspruch 30, wobei die rekombinante Verbindung zur Erzeugung von Thrombin mit zumindest einem Phospholipid kombiniert wird, welches aus der Gruppe ausgewählt wurde, die aus Phosphatidylserin, Derivaten davon, Phosphatidylserin mit zumindest einer Fettsäurenseitenkette, Phosphatidylcholin, Derivaten davon, Phosphatidylcholin mit zumindest einer Fettsäureseitenkette und Mischungen daraus besteht.

36. Das Verfahren gemäß Anspruch 30, wobei die rekombinante Verbindung zur Erzeugung von Thrombin mit zumindest einem Phospholipid kombiniert wird, welches aus der Gruppe ausgewählt wurde, die aus Phosphatidylserin, Derivaten davon, Phosphatidylserin mit zumindest einer Fettsäurenseitenkette, Phosphatidylcholin, Derivaten davon, Phosphatidylcholin mit zumindest einer Fettsäureseitenkette und Mischungen daraus besteht, wobei die Fettsäurenseitenkette aus der Gruppe ausgewählt wurde, welche aus Fettsäureseitenketten mit zumindest einer Doppelbindung und mit 6 bis 24 Kohlenstoffatomen besteht.

37. Das verfahren gemäß Anspruch 30, wobei die rekombinante Verbindung zur Erzeugung von Thrombin mit zumindest einem Phospholipid kombiniert wird, welches aus der Gruppe ausgewählt wurde, die aus Phosphatidylserin, Derivaten davon, Phosphatidylserin mit zumindest einer Fettsäurenseitenkette, Phosphatidylcholin, Derivaten davon, Phosphatidylcholin mit zumindest einer Fettsäureseitenkette und Mischungen daraus besteht, wobei die Fettsäurenseitenkette aus der Gruppe ausgewählt wurde, welche aus Fettsäureseitenketten mit zumindest einer Doppelbindung und mit 16 bis 18 Kohlenstoffatomen besteht.

38. Das Verfahren gemäß Anspruch 30, wobei die rekombinante Verbindung zur Erzeugung von Thrombin mit einer Mischung von zumindest zwei Phospholipiden kombiniert wird, wobei das erste aus der Gruppe ausgewählt wurde, die aus Phosphatidylserin, Derivaten davon, Phosphatidylserin mit zumindest einer Fettsäurenseitenkette und Mischungen daraus besteht, wobei die Fettsäurenseitenkette des Phosphatidylserins zumindest über eine Fettsäurenseitekette verfügt, welche aus der Gruppe ausgewählt wurde, die aus Fettsäureseitenketten mit zumindest einer Doppelbindung und 6 bis 24 Kohlenstoffatomen besteht, während das zweite Phospholipid aus der Gruppe ausgewählt wurde, welche aus Phosphatidylcholin, Derivaten davon, Phosphatidylcholin mit zumindest einer Fettsäureseitenkette und Mischungen davon besteht, wobei die Fettsäurenseitenkette des Phosphatidylcholins zumindest über eine Fettsäureseitenkette verfügt, die aus der Gruppe ausgewählt wurde, die aus Fettsäureseitenketten mit zumindest einer Doppelbindung und mit 6 bis 24 Kohlenstoffatomen besteht.

39. Das Verfahren gemäß Anspruch 30, wobei die kalziumhaltige Verbindung aus der Gruppe ausgewählt wurde, die aus Knochenpartikeln und einer Mischung aus Knochenpartikeln und kalziumhaltigen Additiven besteht und wobei die Knochenpartikel Knochenpartikel sind, die aus der Gruppe ausgewählt wurden, die aus Partikeln des Schädels und des Hüftbeins und aus Mischungen davon besteht.

40. Das Verfahren gemäß Anspruch 30, wobei die kalziumhaltige Verbindung aus der Gruppe ausgewählt wurde, die aus Knochenpartikeln und einer Mischung aus Knochenpartikeln und kalziumhaltigen Additiven besteht und wobei die Knochenpartikel Partikel aus nicht denaturierten Knochen sind.

41. Das Verfahren gemäß Anspruch 30, wobei die kalziumhaltige Verbindung aus der Gruppe ausgewählt wurde, die aus Knochenpartikeln und einer Mischung aus Knochenpartikeln und kalziumhaltigen Additiven besteht und wobei die Knochenpartikel eine durchschnittliche Partikelgröße zwischen 0,5 mm und 5 mm aufweisen.

42. Das Verfahren gemäß Anspruch 30, wobei die kalziumhaltige Verbindung aus der Gruppe ausgewählt wurde, die aus Knochenpartikeln und einer Mischung aus Knochenpartikeln und kalziumhaltigen Additiven besteht und wobei die Anzahl der zum thrombozytenreichen Plasma hinzugegebenen Knochenpartikel 15 bis 50 Volumenprozent der Mischung aus thrombozytenreichem Plasma und Knochenpartikeln entspricht.

43. Das Verfahren gemäß Anspruch 30, wobei die Mischung aus thrombozytenreichem Plasma, Knochenpartikeln und der rekombinanten, Thrombin erzeugenden Verbindung eine Thrombozytenkonzentration von 1.500.000 bis 2.000.000 Thrombozyten pro Mikroliter der Mischung ohne Knochenpartikel hat und 0,05 bis 0,4 *µ*g Thromboplastin in trockener Form pro Mikroliter der Mischung ohne Knochenpartikel enthält.

44. Das Verfahren gemäß Anspruch 30 zur Herstellung eines knochenerzeugenden Produktes für einen Patienten, wobei das thrombozytenreiche Plasma aus einem Plasma hergestellt wurde, das aus der Gruppe ausgewählt wurde, welche aus dem Plasma des Patienten, einem mit dem Patienten hystokompatiblen Plasma und Mischungen davon besteht und wobei die kalziumhaltige Verbindung hauptsächlich aus Knochenpartikeln besteht, die zumindest aus einem Knochen hergestellt wurden, der aus der Gruppe ausgewählt wurde, welche aus Knochen des Patienten, mit dem Patienten hystokompatiblen Knochen und Mischungen davon besteht.

45. Das Verfahren gemäß Anspruch 30, wobei die Koagulation des thrombozytenreichen Plasmas in Anwesenheit vor einem Antibiotikum durchgeführt wird, das vorzugsweise einen antiosteoklastischen Effekt aufweist.

46. Verwendung eines rekombinanten Thromboplastins in einer Mischung aus zumindest einem Phospholipid zur Herstellung eines knochenerzeugenden Produktes aus einer Mischung aus thrombozytenreichem Plasma mit einer Thrombozytenkonzentration von 1.500.000 bis 2.000.000 Thrombozyten pro Mikroliter, einer effektiven Menge einer kalziumhaltigen Verbindung zur Induzierung der Knochenerzeugung und möglicherweise einem Antibiotikum, das vorzugsweise einen antiosteoklastischen Effekt aufweist.

## Revendications

1. Procédé pour la préparation d'un matériau d'obturation, dans lequel un plasma riche en plaquettes ayant une concentration en plaquettes de 1 500 000 à 2 000 000 plaquettes par microlitre, est mis en contact avec une thromboplastine recombinante en présence d'au moins un phospholipide et d'un agent tampon.

2. Procédé selon la revendication 1, dans lequel un gel est formé en mettant en contact un plasma riche en plaquettes ayant une concentration en plaquettes de 1 500 000 à 2 000 000 plaquettes par microlitre, avec un composé recombinant pour générer de la thrombine en présence d'au moins un phospholipide, d'au moins un antibiotique et d'au moins une quantité efficace de tampon, de sorte que le pH du plasma riche en plaquettes mis en contact soit maintenu entre 6 et 8, de manière avantageuse entre 7 et 7,5 au cours de la formation du gel.

3. Procédé selon la revendication 2, dans lequel on prépare une solution tamponnée contenant le ou les antibiotiques et le tampon, éventuellement un composé recombinant pour générer de la thrombine et éventuellement un phospholipide, ladite solution tamponnée ayant un pH compris entre 6 et 8, de préférence entre 7 et 7,5 et dans lequel le plasma riche en plaquettes est mis en contact avec ladite solution tamponnée.

4. Procédé selon l'une quelconque des revendications 1 à 3, dans lequel le plasma riche en plaquettes est mis en contact avec un composé recombinant pour générer de la thrombine en présence d'au moins deux phospholipides différents.

5. Procédé selon l'une quelconque des revendications 1 à 4, dans lequel le plasma riche en plaquettes est mis en contact avec un composé recombinant pour générer de la thrombine en présence d'au moins un phospholipide choisi dans le groupe constitué de la phosphatidylsérine, de ses dérivés, de la phosphatidylsérine ayant au moins une chaîne latérale d'acide gras, de la phosphatidylcholine, de ses dérivés et de la phosphatidylcholine ayant au moins une chaîne latérale d'acide gras, de préférence d'au moins deux phospholipides choisis dans ledit groupe.

6. Procédé selon la revendication 5, dans lequel la chaîne latérale d'acide gras de la phosphatidylcholine est choisie dans le groupe constitué de chaînes d'acides gras avec au moins une double liaison et 6 à 24 atomes de carbone, de préférence 16 à 18 atomes de carbone.

7. Procédé selon les revendications 1 à 6, dans lequel le plasma riche en plaquettes est mis en contact avec au moins un antibiotique ayant un effet anti-ostéoclastique.

8. Procédé selon les revendications 1 à 7, dans lequel le plasma riche en plaquettes est mis en contact avec 0,05 à 0,4 *µ*g de thromboplastine sous forme sèche par microlitre et avec 0,05 à 4 *µ*g d'antibiotique(s) par microlitre en présence d'une quantité efficace d'agent(s) tampon(s) pour réguler le pH entre 6 et 8, de manière avantageuse entre 7 et 7,5 au cours de la gélification.

9. Trousse pour la préparation d'un matériau d'obturation, ladite trousse comprenant :
- un premier flacon contenant un plasma riche en plaquettes ayant une concentration en plaquettes de 1 500 000 à 2 000 000 plaquettes par microlitre;
- un deuxième flacon contenant une thromboplastine recombinante, de préférence une solution contenant ladite thromboplastine recombinante;
- éventuellement, un troisième flacon contenant une solution à ajouter au premier flacon et/ou au deuxième flacon pour la préparation d'un plasma riche en plaquettes ayant une concentration en plaquettes de 1 500 000 à 2 000 000 plaquettes par microlitre et/ou une solution contenant un composé recombinant pour générer de la thrombine;
dans laquelle le premier flacon et/ou le deuxième flacon et/ou le troisième flacon contient ou contiennent au moins un phospholipide, de préférence au moins deux phospholipides différents, et dans laquelle le premier flacon et/ou le deuxième flacon et/ou le troisième flacon, de préférence le deuxième flacon ou le troisième flacon, contient ou contiennent au moins un antibiotique.

10. Trousse selon la revendication 9, dans laquelle le premier flacon et/ou le deuxième flacon et/ou le troisième flacon contient ou contiennent au moins un agent tampon.

11. Trousse selon la revendication 10, dans laquelle le deuxième flacon contient au moins un phospholipide, au moins un agent tampon et au moins un antibiotique.

12. Trousse selon la revendication 9, dans laquelle le premier flacon contient un plasma riche en plaquettes ayant une concentration en plaquettes de 1 500 000 à 2 000 000 plaquettes par microlitre sous une forme sèche, tandis que le deuxième flacon contient un composé recombinant pour générer de la thrombine sous une forme sèche.

13. Produit générateur d'os comprenant une matrice coagulée de plasma riche en plaquettes ayant une concentration en plaquettes de 1 500 000 à 2 000 000 plaquettes par microlitre de l'agent formant la matrice avec une thromboplastine recombinante en présence d'au moins un phospholipide, et une quantité efficace de composé contenant du calcium dispersée dans ladite matrice pour induire la formation d'os.

14. Produit générateur d'os selon la revendication 13, dans lequel le composé contenant du calcium est choisi dans le groupe constitué de particules osseuses, d'un mélange de particules osseuses avec des additifs contenant du calcium.

15. Produit générateur d'os selon la revendication 13, qui comprend en outre au moins deux phospholipides différents.

16. Produit générateur d'os selon la revendication 13, dans lequel le composé recombinant pour générer de la thrombine est combiné avec des phospholipides.

17. Produit générateur d'os selon la revendication 13, dans lequel le composé recombinant pour générer de la thrombine est combiné avec au moins un phospholipide choisi dans le groupe constitué de la phosphatidylsérine, de ses dérivés, de la phosphatidylsérine ayant au moins une chaîne latérale d'acide gras, de la phosphatidylcholine, de ses dérivés, de la phosphatidylcholine ayant au moins une chaîne latérale d'acide gras et de leurs mélanges.

18. Produit générateur d'os selon la revendication 13, dans lequel le composé recombinant pour générer de la thrombine est combiné avec au moins un phospholipide choisi dans le groupe constitué de la phosphatidylsérine, de ses dérivés, de la phosphatidylsérine ayant au moins une chaîne latérale d'acide gras, de la phosphatidylcholine, de ses dérivés, de la phosphatidylcholine ayant au moins une chaîne latérale d'acide gras et de leurs mélanges, la chaîne latérale d'acide gras étant choisie dans le groupe constitué de chaînes d'acides gras avec au moins une double liaison et 6 à 24 atomes de carbone.

19. Produit générateur d'os selon la revendication 13, dans lequel le composé recombinant pour générer de la thrombine est combiné avec au moins un phospholipide choisi dans le groupe constitué de la phosphatidylsérine, de ses dérivés, de la phosphatidylsérine ayant au moins une chaîne latérale d'acide gras, de la phosphatidylcholine, de ses dérivés, de la phosphatidylcholine ayant au moins une chaîne latérale d'acide gras et de leurs mélanges, la chaîne latérale d'acide gras étant choisie dans le groupe constitué de chaînes d'acides gras avec au moins une double liaison et 16 à 18 atomes de carbone.

20. Produit générateur d'os selon la revendication 13, dans lequel le composé recombinant pour générer de la thrombine est combiné avec un mélange d'au moins deux phospholipides, un premier phospholipide étant choisi dans le groupe constitué de la phosphatidylsérine, de ses dérivés, de la phosphatidylsérine ayant au moins une chaîne latérale d'acide gras et de leurs mélanges, la chaîne latérale d'acide gras de la phosphatidylsérine ayant au moins une chaîne latérale d'acide gras choisie dans le groupe constitué de chaînes d'acides gras avec au moins une double liaison et 6 à 24 atomes de carbone, tandis que le second phospholipide est choisi dans le groupe constitué de la phosphatidylcholine, de ses dérivés, de la phosphatidylcholine ayant au moins une chaîne latérale d'acide gras et de leurs mélanges, la chaîne latérale d'acide gras de la phosphatidylcholine ayant au moins une chaîne latérale d'acide gras choisie dans le groupe constitué de chaînes d'acides gras avec au moins une double liaison et 6 à 24 atomes de carbone.

21. Produit générateur d'os selon la revendication 13, dans lequel le composé contenant du calcium est choisi dans le groupe constitué de particules osseuses, d'un mélange de particules osseuses avec des additifs contenant du calcium, et dans lequel les particules osseuses sont des particules osseuses choisies dans le groupe constitué de particules osseuses craniofaciales, de particules osseuses iliaques et de leurs mélanges.

22. Produit générateur d'os selon la revendication 13, dans lequel le composé contenant du calcium est choisi dans le groupe constitué de particules osseuses, d'un mélange de particules osseuses avec des additifs contenant du calcium, et dans lequel les particules osseuses sont des particules d'os non dénaturés.

23. Produit générateur d'os selon la revendication 13, dans lequel les particules osseuses ont une taille particulaire moyenne comprise entre 0, 5 mm et 5 mm.

24. Produit générateur d'os selon la revendication 13, dans lequel le composé contenant du calcium est choisi dans le groupe constitué de particules osseuses, d'un mélange de particules osseuses avec des additifs contenant du calcium, le produit comprenant de 15% à 50% en volume de particules osseuses.

25. Produit générateur d'os selon la revendication 13, dans lequel la matrice coagulée est une matrice coagulée de plasma riche en plaquettes ayant une concentration en plaquettes de 1 500 000 à 2 000 000 plaquettes par microlitre des agents formant la matrice et 0,05 à 0,4 *µ*g de thromboplastine sous forme sèche par microlitre des agents formant la matrice.

26. Produit générateur d'os selon la revendication 13 pour un patient, dans lequel le plasma riche en plaquettes est préparé à partir du plasma du patient et dans lequel les particules osseuses sont préparées à partir d'un os du patient.

27. Produit générateur d'os selon la revendication 13, dans lequel la matrice coagulée est associée à un film biocompatible.

28. Produit générateur d'os selon la revendication 13, qui contient en outre au moins un additif choisi dans le groupe constitué des facteurs de croissance, des gènes codant pour les facteurs de croissance, des composés contenant du calcium, des médicaments, des acides gras, des antibiotiques, des bactéricides, des virucides, du fibrinogène, des composés induisant la formation de matrices et de leurs mélanges.

29. Produit générateur d'os selon la revendication 28, qui contient comme antibiotique au moins un antibiotique ayant un effet anti-ostéoclastique.

30. Procédé de préparation d'un produit générateur d'os comprenant une matrice coagulée de plasma riche en plaquettes ayant une concentration en plaquettes de 1 500 000 à 2 000 000 plaquettes par microlitre de l'agent formant la matrice, avec une thromboplastine recombinante, et des particules osseuses dispersées dans ladite matrice, dans lequel :
- un mélange sensiblement homogène est préparé en mélangeant du plasma riche en plaquettes avec une quantité de composé contenant du calcium efficace pour induire la génération d'os lorsque l'on ajoute un composé recombinant générateur de thrombine et un phospholipide,
- un composé recombinant générateur de thrombine et un phospholipide sont ajoutés et mélangés au mélange préparé à partir de plasma riche en plaquettes, et
- le mélange de composé recombinant générateur de thrombine, de phospholipide, de plasma riche en plaquettes et de composé contenant du calcium est maintenu dans des conditions permettant d'assurer une coagulation du plasma riche en plaquettes et la formation d'une matrice.

31. Procédé selon la revendication 30, dans lequel le composé contenant du calcium est choisi dans le groupe constitué de particules osseuses, d'un mélange de particules osseuses avec des additifs contenant du calcium.

32. Procédé selon la revendication 30, dans lequel la coagulation est effectuée en présence d'oxygène et sensiblement sans agitation.

33. Procédé selon la revendication 30, dans lequel le composé contenant du calcium est choisi dans le groupe constitué de particules osseuses, d'un mélange de particules osseuses avec des additifs contenant du calcium, et dans lequel au moins un phospholipide est ajouté au'mélange choisi dans le groupe constitué d'un mélange de composé recombinant générateur de thrombine, de plasma riche en plaquettes et de particules osseuses et d'un mélange de plasma riche en plaquettes et de particules osseuses.

34. Procédé selon la revendication 30, dans lequel le composé recombinant pour générer de la thrombine est combiné avec des phospholipides.

35. Procédé selon la revendication 30, dans lequel le composé recombinant pour générer de la thrombine est combiné avec au moins un phospholipide choisi dans le groupe constitué de la phosphatidylsérine, de ses dérivés, de la phosphatidylsérine ayant au moins une chaîne latérale d'acide gras, de la phosphatidylcholine, de ses dérivés, de la phosphatidylcholine ayant au moins une chaîne latérale d'acide gras et de leurs mélanges.

36. Procédé selon la revendication 30, dans lequel le composé recombinant pour générer de la thrombine est combiné avec au moins un phospholipide choisi dans le groupe constitué de la phosphatidylsérine, de ses dérivés, de la phosphatidylsérine ayant au moins une chaîne latérale d'acide gras, de la phosphatidylcholine, de ses dérivés, de la phosphatidylcholine ayant au moins une chaîne latérale d'acide gras et de leurs mélanges, la chaîne latérale d'acide gras étant choisie dans le groupe constitué de chaînes d'acides gras avec au moins une double liaison et 6 à 24 atomes de carbone.

37. Procédé selon la revendication 30, dans lequel le composé recombinant pour générer de la thrombine est combiné avec au moins un phospholipide choisi dans le groupe constitué de la phosphatidylsérine, de ses dérivés, de la phosphatidylsérine ayant au moins une chaîne latérale d'acide gras, de la phosphatidylcholine, de ses dérivés, de la phosphatidylcholine ayant au moins une chaîne latérale d'acide gras et de leurs mélanges, la chaîne latérale d'acide gras étant choisie dans le groupe constitué de chaînes d'acides gras avec au moins une double liaison et 16 à 18 atomes de carbone.

38. Procédé selon la revendication 30, dans lequel le composé recombinant pour générer de la thrombine est combiné avec un mélange d'au moins deux phospholipides, un premier phospholipide étant choisi dans le groupe constitué de la phosphatidylsérine, de ses dérivés, de la phosphatidylsérine ayant au moins une chaîne latérale d'acide gras et de leurs mélanges, la chaîne latérale d'acide gras de la phosphatidylsérine ayant au moins une chaîne latérale d'acide gras choisie dans le groupe constitué de chaînes d'acides gras avec au moins une double liaison et 6 à 24 atomes de carbone, tandis que le second phospholipide est choisi dans le groupe constitué de la phosphatidylcholine, de ses dérivés, de la phosphatidylcholine ayant au moins une chaîne latérale d'acide gras et de leurs mélanges, la chaîne latérale d'acide gras de la phosphatidylcholine ayant au moins une chaîne latérale d'acide gras choisie dans le groupe constitué de chaînes d'acides gras avec au moins une double liaison et 6 à 24 atomes de carbone.

39. Procédé selon la revendication 30, dans lequel le composé contenant du calcium est choisi dans le groupe constitué de particules osseuses, d'un mélange de particules osseuses avec des additifs contenant du calcium, et dans lequel les particules osseuses sont des particules osseuses choisies dans le groupe constitué de particules osseuses craniofaciales, de particules osseuses iliaques et de leurs mélanges.

40. Procédé selon la revendication 30, dans lequel le composé contenant du calcium est choisi dans le groupe constitué de particules osseuses, d'un mélange de particules osseuses avec des additifs contenant du calcium, et dans lequel les particules osseuses sont des particules d'os non dénaturés.

41. Procédé selon la revendication 30, dans lequel le composé contenant du calcium est choisi dans le groupe constitué de particules osseuses, d'un mélange de particules osseuses avec des additifs contenant du calcium, et dans lequel les particules osseuses ont une taille particulaire moyenne comprise entre 0,5 mm et 5 mm.

42. Procédé selon la revendication 30, dans lequel le composé contenant du calcium est choisi dans le groupe constitué de particules osseuses, d'un mélange de particules osseuses avec des additifs contenant du calcium, et dans lequel la quantité de particules osseuses ajoutée au plasma riche en plaquettes correspond à environ 15% à 50% en volume du mélange de plasma riche en plaquettes et de particules osseuses.

43. Procédé selon la revendication 30, dans lequel le mélange de plasma riche en plaquettes, de particules osseuses et de composé recombinant générateur de thrombine a une concentration en plaquettes de 1 500 000 à 2 000 000 plaquettes par microlitre du mélange sans particules osseuses et contient 0,05 à 0,4 *µ*g de thromboplastine sous forme sèche par microlitre du mélange sans particules osseuses.

44. Procédé selon la revendication 30 pour la préparation d'un produit générateur d'os pour un patient, dans lequel le plasma riche en plaquettes est préparé à partir d'un plasma choisi dans le groupe constitué du plasma du patient, d'un plasma hystocompatible avec le patient et de leurs mélanges, et dans lequel le composé contenant du calcium est constitué essentiellement de particules osseuses préparées à partir d'au moins un os choisi dans le groupe constitué des os du patient, des os hystocompatibles avec le patient et de leurs mélanges.

45. Procédé selon la revendication 30, dans lequel la coagulation du plasma riche en plaquettes se fait en présence d'un antibiotique, ayant de préférence un effet anti-ostéoclastique.

46. Utilisation d'une thromboplastine recombinante en mélange avec au moins un phospholipide pour la préparation d'un produit générateur d'os à partir d'un mélange d'un plasma riche en plaquettes, ayant une concentration en plaquettes de 1 500 000 à 2 000 000 plaquettes par microlitre, une quantité efficace de composé contenant du calcium pour induire une génération d'os et, éventuellement, un antibiotique ayant de préférence un effet anti-ostéoclastique.
